# EUROPEAN PATENT SPECIFICATION

(11) **EP 3 148 495 B1**
(45) Date of publication and mention of the grant of the patent: **15.07.2020**
(21) Application number: 15726840.0
(22) Date of filing: 20.05.2015
(51) Int. Cl.: A61F 13/15

(54) **METHOD OF MAKING AN ABSORBENT PRODUCT**
VERFAHREN ZUR HERSTELLUNG VON SAUGFÄHIGEN PRODUKTEN
PROCÉDÉ DE FABRICATION D'UN PRODUIT ABSORBANT

(30) Priority: 27.05.2014 US 201462003090 P
(43) Date of publication of application: 05.04.2017
(73) Proprietor: The Procter & Gamble Company, Cincinnati, OH 45202 (US)
(72) Inventor: ARNOLD, James R., Cincinnati, Ohio 45202 (US)
(74) Representative: P&G Patent Germany
(86) International application number: PCT/US2015/031684
(87) International publication number: WO 2015/183645

(56) References cited:
- EP-A1- 2 604 237
- US-A1- 2006 138 010
- US-A1- 2013 130 879
- US-A1- 2013 130 880

## Description

### FIELD OF THE INVENTION

The present disclosure relates to methods for making absorbent products including packages of folded absorbent articles, and more particularly, methods for folding absorbent articles and placing the folded absorbent articles into stacker receptacles.

### BACKGROUND OF THE INVENTION

Along an assembly line, various types of articles, such as for example, diapers and other absorbent articles, may be assembled by adding components to and otherwise modifying an advancing, continuous web of material. For example, in some processes, advancing webs of material are combined with other advancing webs of material. In other examples, individual components created from advancing webs of material are combined with advancing webs of material, which in turn, are then combined with other advancing webs of material. Webs of material and component parts used to manufacture diapers may include: backsheets, topsheets, leg cuffs, waist caps, absorbent core components, front and/or back ears, fastener components, and various types of elastic webs and components such as leg elastics, barrier leg cuff elastics, stretch side panels, and waist elastics. Once the desired component parts are assembled, the advancing web(s) and component parts are subjected to a final knife cut to separate the web(s) into discrete diapers or other absorbent articles. After the final knife cut, absorbent articles may also undergo a folding process prior to packaging.

Diaper pants may also include additional manufacturing steps not used in the manufacture of conventional taped diapers. For example, diaper pants may include a front elastic belt and a rear elastic belt connected with opposing longitudinal end regions of a chassis that includes an absorbent core. Thus, after folding the chassis into a U-shape about a lateral centerline, opposing lateral end regions of the elastic belts may be connected with each other at side seams to form a diaper pant having a waist opening and a pair of leg openings. When manufacturing relatively large sized diaper pants, such as adult incontinence products, it may be desirable to further fold various components of the diaper pants in some manner to allow for a relatively better fit within a package.

In some manufacturing processes, diaper pants may advance through apparatuses that fold portions of the elastic belts and/or chassis in various ways to provide a relatively compact and neatly folded diaper pant. The folded diaper pants may then advance from the folding operations to a stacking apparatus that collects and arranges the folded diapers into stacks. And from the stacking apparatus, stacks of folded diapers may advance to a packaging apparatus that places stacks of folded diapers into packages.

Relatively large sizes of diaper pants in combination with relatively high manufacturing speeds may present challenges in precisely controlling areas of the products during folding, stacking, and packing processes. For example, diaper pants may be manufactured at relatively high rates of speed, and as such, the diaper pants advance rapidly through various folding operations and placed into stacks before being transferred into packages. During such rapid folding and transferring operations, forces exerted on the diaper pants as a result of the relatively high travel speeds and friction from unit operation apparatuses in combination with elasticity of the diaper pant components may result inconsistently folded diaper pants. In some operations, folded portions of the diaper pants may not remain in desired positions and/or may not be folded completely as intended. As such, during the folding, stacking, and packaging processes, some portions of a folded diaper pant may remain and/or may become extended outward from what would otherwise be a relatively neatly folded and compact article. In turn, such extended portions can cause the folded diaper pants to jam while advancing from folding, stacking, and/or packing operations. In some configurations, folded diapers may advance across a gap located between a folding apparatus conveyor to a stacking apparatus platform. While advancing across such a gap, extended or improperly folded portions of folded articles may cause the folded articles to shift, jam, and/or otherwise interfere with stacking and packaging operations. In turn, improperly packaged articles, unintended line shutdowns, and/or process equipment damage may result.

Consequently, it would be beneficial to provide methods for manufacturing absorbent articles to more consistently produce neatly folded articles. It would also be beneficial to arrange the sequence of certain operations to take advantage of various forces exerted on absorbent articles during folding operations so as to create, maintain, and position tightly folded areas of articles in desired positions relative to folding and stacking apparatuses as articles are rapidly transferred from folding processes to stacking and packaging operations.

US 2013/130879 discloses a method of folding a pant-like disposable absorbent garment. The method includes providing a garment defining first and second waist side regions, a waist center region positioned therebetween, and a crotch region longitudinally below the waist center region. The method may further include folding the garment along a transversely extending fold line so as to bring the crotch region into superposed relation with the waist center region; providing a trough having a floor and first and second side walls; placing the waist center region in the trough; and, while the garment is in the trough, folding the garment along longitudinally extending first and second fold lines so as to position the first and second waist side regions, respectively, over the waist center region. The first and second fold lines are adjacent the first and second side walls, respectively.

US 2013/130880 relates to a method of folding a pant-like disposable absorbent garment. The method includes providing a garment defining first and second waist side regions, a waist center region positioned therebetween, and a crotch region longitudinally below the waist center region. The method may further include folding the garment along a transversely extending fold line so as to bring the crotch region into superposed relation with the waist center region; providing a chute having a floor and first and second side walls; urging the garment into the chute; and, while the garment is in the chute, folding the garment along longitudinally extending first and second fold lines so as to position the first and second waist side regions over the waist center region. The first and second fold lines are adjacent the first and second side walls, respectively.

EP 2604237 A1 relates to an apparatus for producing arrays of absorbent articles with varying orientations. A method for forming alternating arrays of absorbent articles, suitable to be operated at a high speed, is also provided.

### SUMMARY OF THE INVENTION

The present disclosure relates to methods and apparatuses for folding absorbent articles and placing such folded absorbent articles into stacker receptacles. The absorbent articles are in the form of diaper pants that are advanced through a folding apparatus and subsequently advanced to a stacker apparatus having stacker receptacles before being packaged. During the folding process, portions of the diaper pants may be subjected to forces to help maintain relatively well defined fold lines in the diaper pants. The relatively well defined fold lines of the folded diaper pants are oriented to promote a relatively smooth transfer from the folding apparatus to the stacker receptacles.

In one embodiment, a method for making an absorbent product comprising a package of absorbent articles, each absorbent article having a longitudinal axis and a lateral axis and comprising: a first elastic belt, a second elastic belt, and a chassis extending longitudinally between the first elastic belt and the second elastic belt; wherein the chassis comprises a topsheet, a backsheet, and an absorbent core disposed between the topsheet and the backsheet; wherein the first and second elastic belts each comprise a first end region and a second end region separated by a central region; and wherein the first end region of the first elastic belt is connected with the first end region of the second elastic belt at a first side seam, and the second end region of the first elastic belt is connected with the second end region of the second elastic belt at a second side seam to define a continuous perimeter waist opening and two continuous perimeter leg openings, wherein the first elastic belt defines a first waist region, the second elastic belt defines a second waist region, the first and second waist regions connected with each other by a crotch region, the method comprising the steps of: folding the absorbent article along a longitudinally extending first fold line to position the first end region of the first elastic belt in a facing relationship with the central region of the first elastic belt; folding the absorbent article along a longitudinally extending second fold line to position the second end region of the first elastic belt in a facing relationship with the first end region of the second elastic belt; forcing the second end region of the second elastic belt across the central region of the first elastic belt; folding the absorbent article along a laterally extending third fold line to position the crotch region in a facing relationship with the first end region of the second elastic belt and the second end region of the second elastic belt to form a folded absorbent article; orienting the folded absorbent article such that the second fold line is below the first fold line; advancing the folded absorbent article in a machine direction to a stacker receptacle, the stacker receptacle comprising: a bottom wall, a rear wall, a first side member, and a second side member spaced apart from the first side member in a cross direction, wherein the first side member and the second side member are both connected with the rear wall; inserting the folded absorbent article into the stacker receptacle such that the second fold line is in contact with the bottom wall, and wherein the central region of the second elastic belt is in a facing relationship with the first side member and wherein the crotch region is in a facing relationship with second side member, wherein the first fold line is above the second fold line, and wherein the third fold line (904) is placed in contact with the rear wall (806) of the receptacle (802); moving the first side member, the second side member, and the folded absorbent article in the cross direction; and advancing the folded absorbent article from the receptacle to the package.

### BRIEF DESCRIPTION OF THE DRAWINGS

Figure 1A is a front perspective view of a diaper pant.
Figure 1B is a rear perspective view of a diaper pant.
Figure 2 is a partially cut away plan view of the diaper pant shown in Figures 1A and 1B in a flat, uncontracted state.
Figure 3A is a cross-sectional view of the diaper pant of Figures 2 and 5 taken along line 3A-3A.
Figure 3B is a cross-sectional view of the diaper pant of Figure 2 and 5 taken along line 3B-3B.
Figure 4 is a front perspective view of a diaper pant constructed with an outer cover.
Figure 5 is a partially cut away plan view of the diaper pant shown in Figure 4 in a flat, uncontracted state.
Figure 6A is a front plan view of a diaper pant.
Figure 6B is a rear plan view of a diaper pant.
Figure 7 is a schematic side view of a folding apparatus and a stacker apparatus.
Figure 8A is a view of the folding apparatus from Figure 7 taken along line 8A-8A.

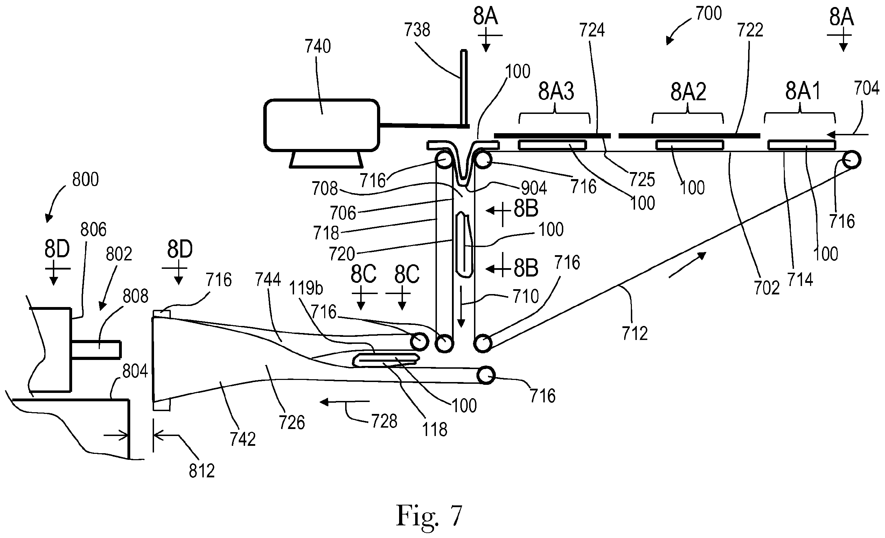

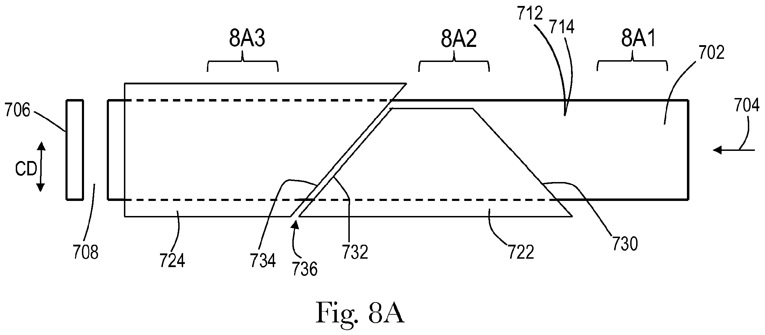

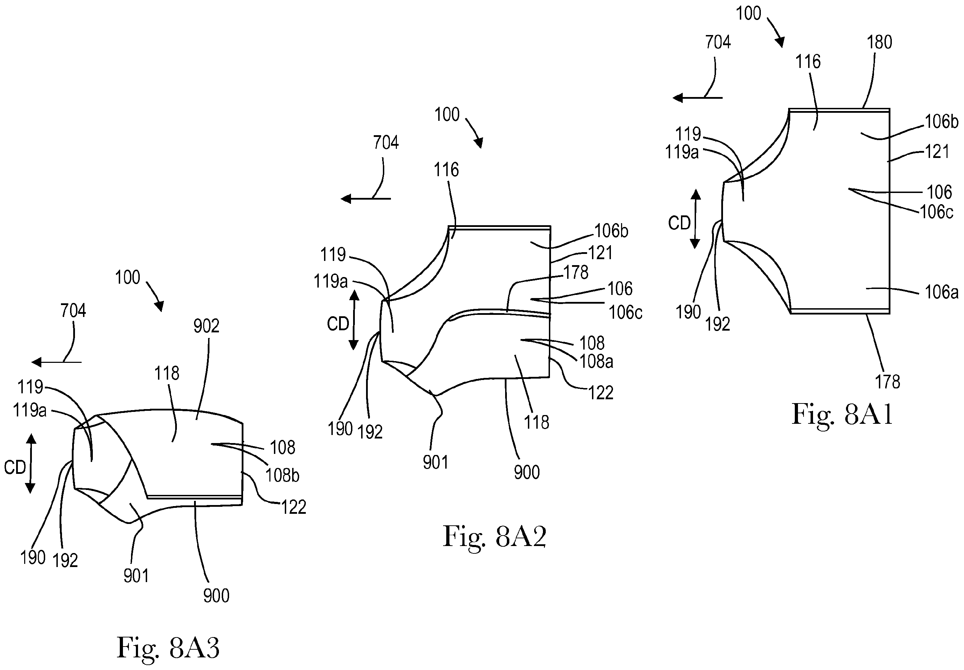

Figure 8A1 is a view a diaper pant advancing through the folding apparatus from Figure 7 taken along line 8A-8A at location 8A1.
Figure 8A2 is a view a diaper pant advancing through the folding apparatus from Figure 7 taken along line 8A-8A at location 8A2.
Figure 8A3 is a view a diaper pant advancing through the folding apparatus from Figure 7 taken along line 8A-8A at location 8A3.

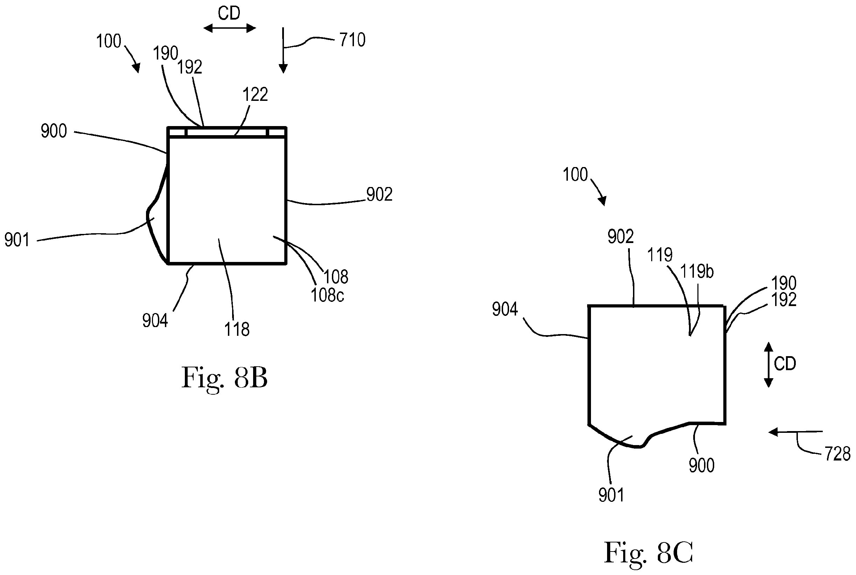

Figure 8B is a view a folded diaper pant advancing through the folding apparatus from Figure 7 taken along line 8B-8B.
Figure 8C is a view a folded diaper pant advancing through the folding apparatus from Figure 7 taken along line 8C-8C.

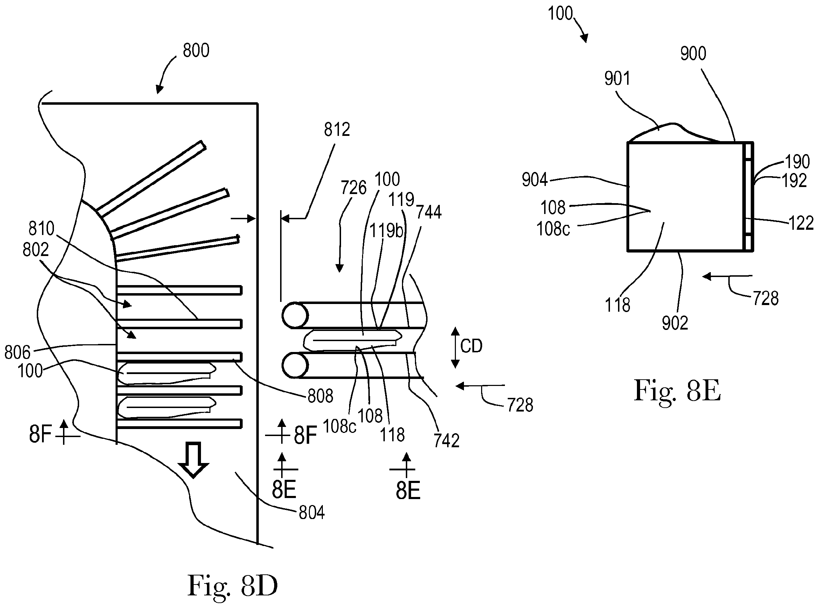

Figure 8D is a view a folded diaper pant advancing through the folding apparatus to a stacker apparatus from Figure 7 taken along line 8D-8D.
Figure 8E is a view a folded diaper pant advancing through the folding apparatus to a stacker apparatus from Figure 8D taken along line 8E-8E.

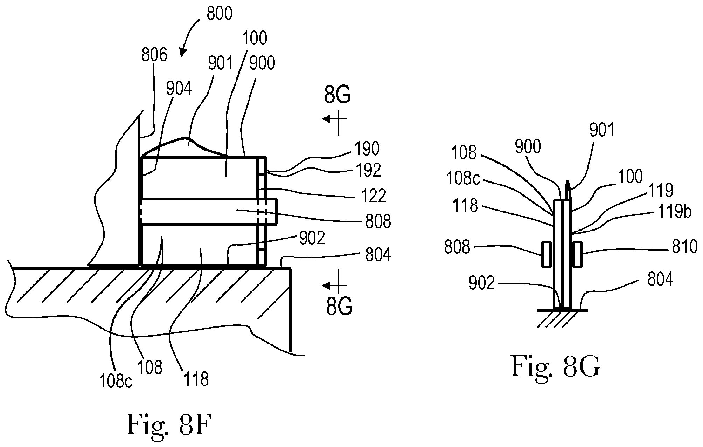

Figure 8F is a view a folded diaper pant positioned in a stacker receptacle from Figure 8D taken along line 8F-8F.
Figure 8G is a view a folded diaper pant positioned in a stacker receptacle from Figure 8F taken along line 8G-8G.

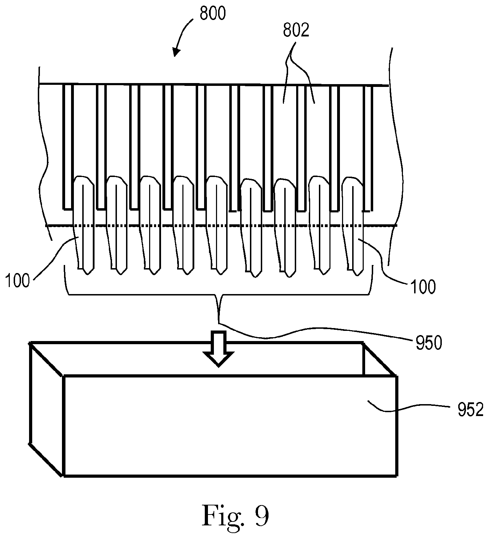

Figure 9 is a view of a stack of folded diaper pants advancing from a stacker apparatus to a package.

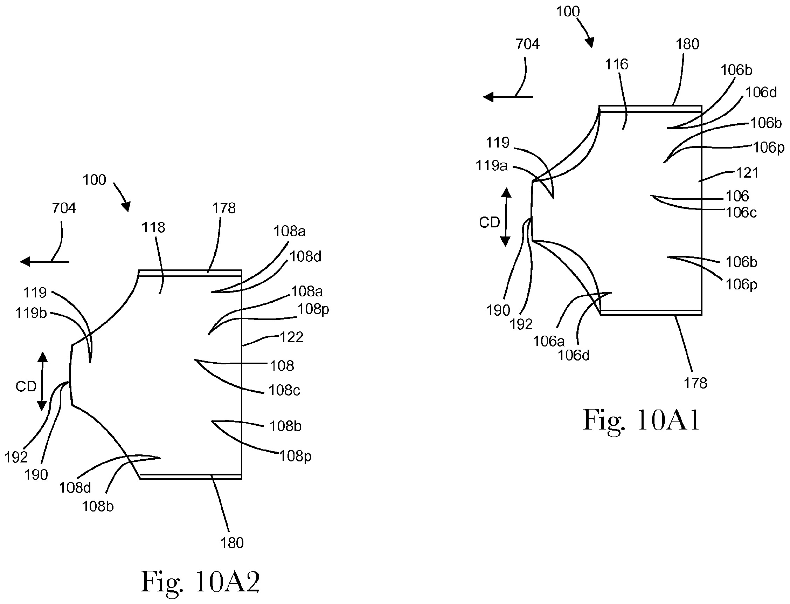

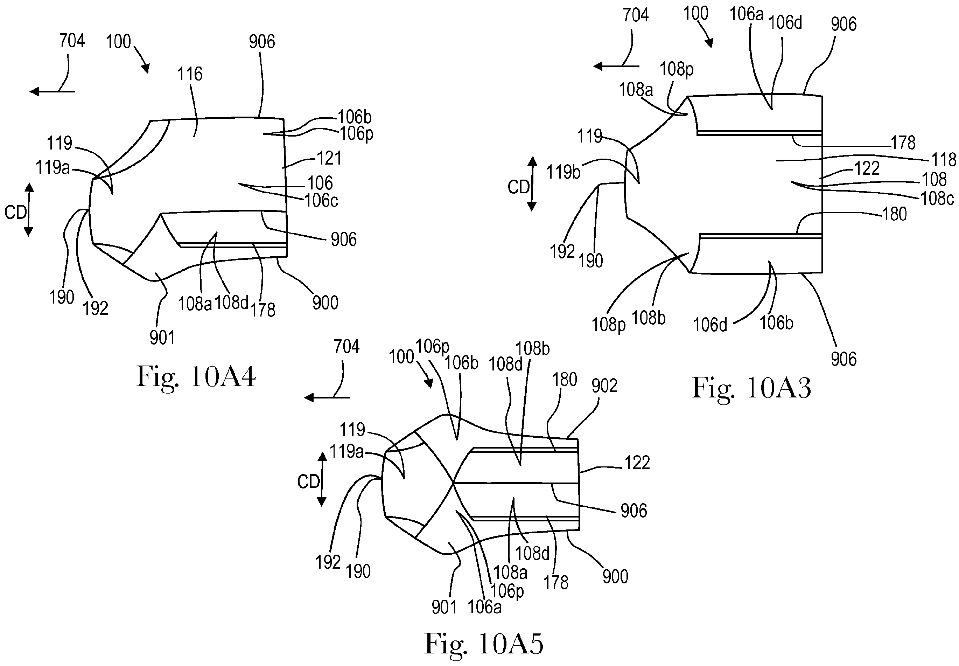

Figure 10A1 is a front plan view a diaper pant advancing through a folding apparatus.
Figure 10A2 is a rear plan view of the diaper pant from Figure 10A1.
Figure 10A3 is a rear view of the diaper pant after being partially folded from the configuration shown in Figure 10A2.
Figure 10A4 is a front view the diaper pant after being further folded from the configuration shown in Figure 10A3.
Figure 10A5 is a front view the diaper pant after being further folded from the configuration shown in Figure 10A4.

### DETAILED DESCRIPTION OF THE INVENTION

The following term explanations may be useful in understanding the present disclosure:
"Absorbent article" is used herein to refer to consumer products whose primary function is to absorb and retain soils and wastes. "Diaper" is used herein to refer to an absorbent article generally worn by infants and incontinent persons about the lower torso. The term "disposable" is used herein to describe absorbent articles which generally are not intended to be laundered or otherwise restored or reused as an absorbent article (e.g., they are intended to be discarded after a single use and may also be configured to be recycled, composted or otherwise disposed of in an environmentally compatible manner).
An "elastic," "elastomer" or "elastomeric" refers to materials exhibiting elastic properties, which include any material that upon application of a force to its relaxed, initial length can stretch or elongate to an elongated length more than 10% greater than its initial length and will substantially recover back to about its initial length upon release of the applied force.
As used herein, the term "joined" encompasses configurations whereby an element is directly secured to another element by affixing the element directly to the other element, and configurations whereby an element is indirectly secured to another element by affixing the element to intermediate member(s) which in turn are affixed to the other element.
"Longitudinal" means a direction running substantially perpendicular from a waist edge to a longitudinally opposing waist edge of an absorbent article when the article is in a flat out, uncontracted state, or from a waist edge to the bottom of the crotch, i.e. the fold line, in a bi-folded article. Directions within 45 degrees of the longitudinal direction are considered to be "longitudinal." "Lateral" refers to a direction running from a longitudinally extending side edge to a laterally opposing longitudinally extending side edge of an article and generally at a right angle to the longitudinal direction. Directions within 45 degrees of the lateral direction are considered to be "lateral."
The term "substrate" is used herein to describe a material which is primarily two-dimensional (i.e. in an XY plane) and whose thickness (in a Z direction) is relatively small (i.e. 1/10 or less) in comparison to its length (in an X direction) and width (in a Y direction). Non-limiting examples of substrates include a web, layer or layers or fibrous materials, nonwovens, films and foils such as polymeric films or metallic foils. These materials may be used alone or may comprise two or more layers laminated together. As such, a web is a substrate.
The term "nonwoven" refers herein to a material made from continuous (long) filaments (fibers) and/or discontinuous (short) filaments (fibers) by processes such as spunbonding, meltblowing, carding, and the like. Nonwovens do not have a woven or knitted filament pattern.
The term "machine direction" (MD) is used herein to refer to the direction of material flow through a process. In addition, relative placement and movement of material can be described as flowing in the machine direction through a process from upstream in the process to downstream in the process.
The term "cross direction" (CD) is used herein to refer to a direction that is generally perpendicular to the machine direction.
The term "pant" (also referred to as "training pant", "pre-closed diaper", "diaper pant", "pant diaper", and "pull-on diaper") refers herein to disposable absorbent articles having a continuous perimeter waist opening and continuous perimeter leg openings designed for adult wearers or infant wearers. A pant can be configured with a continuous or closed waist opening and at least one continuous, closed, leg opening prior to the article being applied to the wearer. A pant can be preformed by various techniques including, but not limited to, joining together portions of the article using any refastenable and/or permanent closure member (e.g., seams, heat bonds, pressure welds, adhesives, cohesive bonds, mechanical fasteners, etc.). A pant can be preformed anywhere along the circumference of the article in the waist region (e.g., side fastened or seamed, front waist fastened or seamed, rear waist fastened or seamed).

The present disclosure relates to methods and apparatuses for assembling absorbent products including packages of absorbent articles. In particular, the present disclosure relates to methods and apparatuses for folding absorbent articles and placing such folded absorbent articles into stacker receptacles. The absorbent articles are in the form of diaper pants, each having a longitudinal axis and a lateral axis. The diaper pants also include a first elastic belt, a second elastic belt, and a chassis, wherein the chassis extends longitudinally between the first elastic belt and the second elastic belt. The chassis includes a topsheet, a backsheet, and an absorbent core disposed between the topsheet and the backsheet. The first and second elastic belts each comprise a first end region and a second end region separated by a central region. The first end region of the first elastic belt is connected with the first end region of the second elastic belt at a first side seam, and the second end region of the first elastic belt is connected with the second end region of the second elastic belt at a second side seam to define a continuous perimeter waist opening and two continuous perimeter leg openings, wherein the first elastic belt defines a first waist region, the second elastic belt defines a second waist region, with the first and second waist regions connected with each other by a crotch region. As discussed in more detail below, assembled diaper pants may be advanced through a folding apparatus and subsequently advanced to a stacker apparatus having stacker receptacles before being packaged. During the folding process, portions of the diaper pants may be subjected to forces to help maintain relatively well defined fold lines in the diaper pants. As the diaper pants advance through the folding process, the relatively well defined fold lines of the folded diaper pants are oriented to promote a relatively smooth transfer from the folding apparatus to the stacker receptacles.

As discussed in more detail below, in some folding apparatus configurations, each absorbent article is folded along longitudinally extending first fold line to position the first end region of the first elastic belt in a facing relationship with the central region of the first elastic belt. Next, the absorbent article is folded along a longitudinally extending second fold line to position the second end region of the first elastic belt in a facing relationship with the first end region of the second elastic belt. The second end region of the second elastic belt is then pulled across the central region of the first elastic belt. As discussed in more detail below, pulling the second end region of the second belt across the central region of the first elastic belt helps to maintain a relatively well defined and smooth second fold line. Subsequently, the absorbent article is folded along a laterally extending third fold line to position the crotch region in a facing relationship with the first end region of the second elastic belt and the second end region of the second elastic belt to form a folded absorbent article. The folded absorbent article is then oriented such that the second fold line is below the first fold line. The folded absorbent article then advances in a machine direction across a gap to between the folding apparatus and a stacker receptacle. The stacker receptacle includes: a bottom wall, a rear wall, a first side member, and a second side member spaced apart from the first side member in a cross direction, wherein the first side member and the second side member are both connected with the rear wall. The folded absorbent article is then inserted into the stacker receptacle such that the second fold line is in contact with the bottom wall. Thus, having the folded absorbent article oriented such that the second longitudinal fold line advances across the gap between the folding and stacking apparatuses helps to allow for a smooth transition from the folding apparatus to the stacker apparatus.

As previously mentioned, the processes and apparatuses discussed herein may be used in the manufacture of different types of absorbent articles. To help provide additional context to the subsequent discussion of the process embodiments, the following provides a general description of absorbent articles in the form of diaper pants that may be folded and stacked in accordance with the methods and apparatuses disclosed herein.

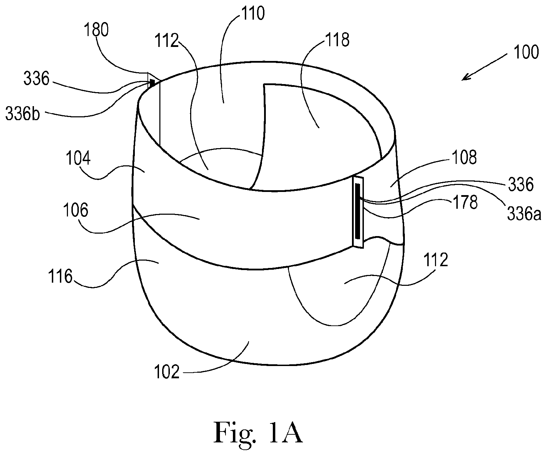

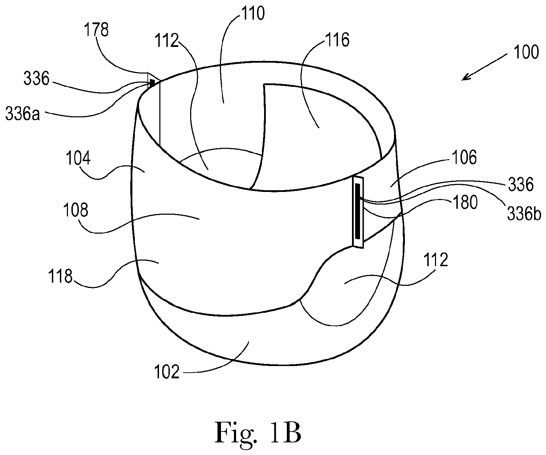

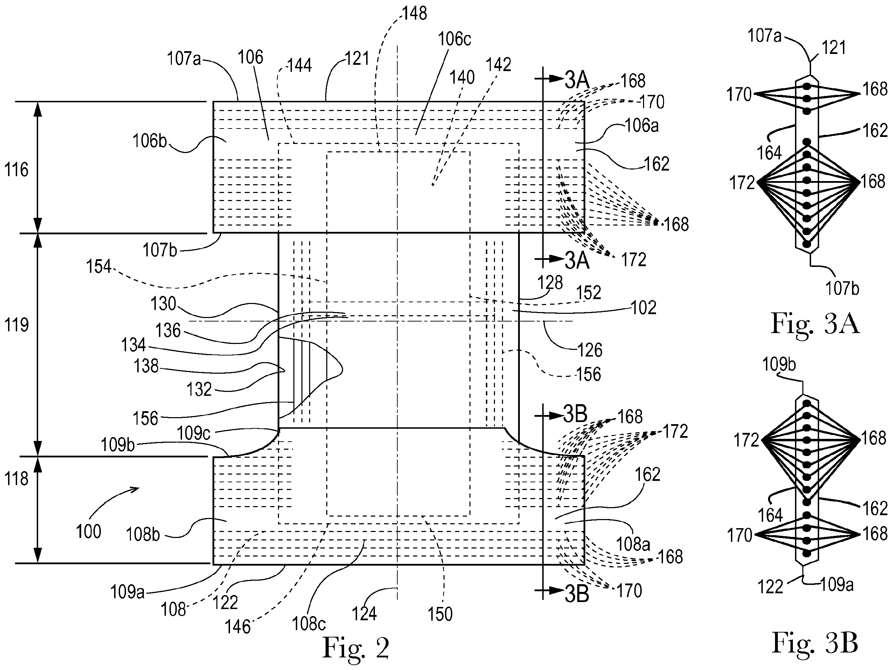

Figures 1A, 1B, and 2 show an example of an absorbent article in the form of a diaper pant 100 that may be assembled and folded and stacked in accordance with the apparatuses and methods disclosed herein. In particular, Figures 1A and 1B show perspective views of a diaper pant 100 in a pre-fastened configuration, and Figure 2 shows a plan view of the diaper pant 100 with the portion of the diaper that faces away from a wearer oriented toward the viewer. The diaper pant 100 includes a chassis 102 and a ring-like elastic belt 104. As discussed below in more detail, a first elastic belt 106 and a second elastic belt 108 are bonded together to form the ring-like elastic belt 104. Although only the second elastic belt 108 is shown with a contoured or shaped edge, it is to be appreciated that either or both the first elastic belt 106 and second elastic belt 108 may include contoured edges.

With continued reference to Figure 2, the diaper pant 100 includes a first waist region 116, a second waist region 118, and a crotch region 119 disposed intermediate the first and second waist regions. The first waist region 116 may be configured as a front waist region, and the second waist region 118 may be configured as back waist region. The diaper 100 may also include a laterally extending front waist edge 121 in the front waist region 116 and a longitudinally opposing and laterally extending back waist edge 122 in the back waist region 118. To provide a frame of reference for the present discussion, the diaper 100 and chassis 102 of Figure 2 is shown with a longitudinal axis 124 and a lateral axis 126. In some embodiments, the longitudinal axis 124 may extend through the front waist edge 121 and through the back waist edge 122. And the lateral axis 126 may extend through a first longitudinal or right side edge 128 and through a midpoint of a second longitudinal or left side edge 130 of the chassis 102.

As shown in Figures 1A, 1B, and 2, the diaper pant 100 may include an inner, body facing surface 132, and an outer, garment facing surface 134. The chassis 102 includes a backsheet 136 and a topsheet 138. The chassis 102 may also include an absorbent assembly 140, including an absorbent core 142, disposed between a portion of the topsheet 138 and the backsheet 136. As discussed in more detail below, the diaper 100 may also include other features, such as leg elastics and/or leg cuffs to enhance the fit around the legs of the wearer.

As shown in Figure 2, the periphery of the chassis 102 may be defined by the first longitudinal side edge 128, a second longitudinal side edge 130, a first laterally extending end edge 144 disposed in the first waist region 116, and a second laterally extending end edge 146 disposed in the second waist region 118. Both side edges 128 and 130 extend longitudinally between the first end edge 144 and the second end edge 146. As shown in Figure 2, the laterally extending end edges 144 and 146 are located longitudinally inward from the laterally extending front waist edge 121 in the front waist region 116 and the laterally extending back waist edge 122 in the back waist region 118. When the diaper pant 100 is worn on the lower torso of a wearer, the front waist edge 121 and the back waist edge 122 of the chassis 102 may encircle a portion of the waist of the wearer. At the same time, the chassis side edges 128 and 130 may encircle at least a portion of the legs of the wearer. And the crotch region 119 may be generally positioned between the legs of the wearer with the absorbent core 142 extending from the front waist region 116 through the crotch region 119 to the back waist region 118.

It is to also be appreciated that a portion or the whole of the diaper 100 may also be made laterally extensible. The additional extensibility may help allow the diaper 100 to conform to the body of a wearer during movement by the wearer. The additional extensibility may also help, for example, the user of the diaper 100, including a chassis 102 having a particular size before extension, to extend the front waist region 116, the back waist region 118, or both waist regions of the diaper 100 and/or chassis 102 to provide additional body coverage for wearers of differing size, i.e., to tailor the diaper to an individual wearer. Such extension of the waist region or regions may give the absorbent article a generally hourglass shape, so long as the crotch region is extended to a relatively lesser degree than the waist region or regions, and may impart a tailored appearance to the article when it is worn.

As previously mentioned, the diaper pant 100 includes a backsheet 136. The backsheet 136 may also define the outer surface 134 of the chassis 102. The backsheet 136 may be impervious to fluids (e.g., menses, urine, and/or runny feces) and may be manufactured in part from a thin plastic film, although other flexible liquid impervious materials may also be used. The backsheet 136 may prevent the exudates absorbed and contained in the absorbent core from wetting articles which contact the diaper 100, such as bedsheets, pajamas and undergarments. The backsheet 136 may also comprise a woven or nonwoven material, polymeric films such as thermoplastic films of polyethylene or polypropylene, and/or a multi-layer or composite materials comprising a film and a nonwoven material (e.g., having an inner film layer and an outer nonwoven layer). The backsheet may also comprise an elastomeric film. An example backsheet 136 may be a polyethylene film having a thickness of from about 0.012 mm (0.5 mils) to about 0.051 mm (2.0 mils). Exemplary polyethylene films are manufactured by Clopay Corporation of Cincinnati, Ohio, under the designation BR-120 and BR-121 and by Tredegar Film Products of Terre Haute, Ind., under the designation XP-39385. The backsheet 136 may also be embossed and/or matte-finished to provide a more clothlike appearance. Further, the backsheet 136 may permit vapors to escape from the absorbent core (i.e., the backsheet is breathable) while still preventing exudates from passing through the backsheet 136. The size of the backsheet 136 may be dictated by the size of the absorbent core 142 and/or particular configuration or size of the diaper 100.

Also described above, the diaper pant 100 includes a topsheet 138. The topsheet 138 may also define all or part of the inner surface 132 of the chassis 102. The topsheet 138 may be compliant, soft feeling, and non-irritating to the wearer's skin. It may be elastically stretchable in one or two directions. Further, the topsheet 138 may be liquid pervious, permitting liquids (e.g., menses, urine, and/or runny feces) to penetrate through its thickness. A topsheet 138 may be manufactured from a wide range of materials such as woven and nonwoven materials; apertured or hydroformed thermoplastic films; apertured nonwovens, porous foams; reticulated foams; reticulated thermoplastic films; and thermoplastic scrims. Woven and nonwoven materials may comprise natural fibers such as wood or cotton fibers; synthetic fibers such as polyester, polypropylene, or polyethylene fibers; or combinations thereof. If the topsheet 138 includes fibers, the fibers may be spunbond, carded, wet-laid, meltblown, hydroentangled, or otherwise processed as is known in the art.

Topsheets 138 may be selected from high loft nonwoven topsheets, apertured film topsheets and apertured nonwoven topsheets. Apertured film topsheets may be pervious to bodily exudates, yet substantially non-absorbent, and have a reduced tendency to allow fluids to pass back through and rewet the wearer's skin. Exemplary apertured films may include those described in U.S. Patent Nos. 5,628,097; 5,916,661; 6,545,197; and 6,107,539.

As mentioned above, the diaper pant 100 may also include an absorbent assembly 140 that is joined to the chassis 102. As shown in Figure 2, the absorbent assembly 140 may have a laterally extending front edge 148 in the front waist region 116 and may have a longitudinally opposing and laterally extending back edge 150 in the back waist region 118. The absorbent assembly may have a longitudinally extending right side edge 152 and may have a laterally opposing and longitudinally extending left side edge 154, both absorbent assembly side edges 152 and 154 may extend longitudinally between the front edge 148 and the back edge 150. The absorbent assembly 140 may additionally include one or more absorbent cores 142 or absorbent core layers. The absorbent core 142 may be at least partially disposed between the topsheet 138 and the backsheet 136 and may be formed in various sizes and shapes that are compatible with the diaper. Exemplary absorbent structures for use as the absorbent core of the present disclosure are described in U.S. Patent Nos. 4,610,678; 4,673,402; 4,888,231; and 4,834,735.

Some absorbent core embodiments may comprise fluid storage cores that contain reduced amounts of cellulosic airfelt material. For instance, such cores may comprise less than about 40%, 30%, 20%, 10%, 5%, or even 1% of cellulosic airfelt material. Such a core may comprises primarily absorbent gelling material in amounts of at least about 60%, 70%, 80%, 85%, 90%, 95%, or even about 100%, where the remainder of the core comprises a microfiber glue (if applicable). Such cores, microfiber glues, and absorbent gelling materials are described in U.S. Patent Nos. 5,599,335; 5,562,646; 5,669,894; and 6,790,798 as well as U.S. Patent Publication Nos. 2004/0158212 and 2004/0097895.

As previously mentioned, the diaper 100 may also include elasticized leg cuffs 156. It is to be appreciated that the leg cuffs 156 can be and are sometimes also referred to as leg bands, side flaps, barrier cuffs, elastic cuffs or gasketing cuffs. The elasticized leg cuffs 156 may be configured in various ways to help reduce the leakage of body exudates in the leg regions. Example leg cuffs 156 may include those described in U.S. Patent Nos. 3,860,003; 4,909,803; 4,695,278; 4,795,454; 4,704,115; 4,909,803; and U.S. Patent Publication Nos. 2009/0312730 A1 and 2013/0255865 A1.

As mentioned above, diaper pants may be manufactured with a ring-like elastic belt 104 and provided to consumers in a configuration wherein the front waist region 116 and the back waist region 118 are connected to each other as packaged, prior to being applied to the wearer. As such, diaper pants may have a continuous perimeter waist opening 110 and continuous perimeter leg openings 112 such as shown in Figures 1A and 1B. The ring-like elastic belt may be formed by joining the first elastic belt to a second elastic belt with a permanent side seam or with an openable and reclosable fastening system disposed at or adjacent the laterally opposing sides of the belts.

As previously mentioned, the ring-like elastic belt 104 is defined by a first elastic belt 106 connected with a second elastic belt 108. As shown in Figure 2, the first elastic belt 106 defines first and second opposing end regions 106a, 106b and a central region 106c, and the second elastic 108 belt defines first and second opposing end regions 108a, 108b and a central region 108c. The central region 106c of the first elastic belt is connected with the first waist region 116 of the chassis 102, and the central region 108c of the second elastic belt 108 is connected with the second waist region 116 of the chassis 102. As shown in Figures 1A and 1B, the first end region 106a of the first elastic belt 106 is connected with the first end region 108a of the second elastic belt 108 at first side seam 178, and the second end region 106b of the first elastic belt 106 is connected with the second end region 108b of the second elastic belt 108 at second side seam 180 to define the ring-like elastic belt 104 as well as the waist opening 110 and leg openings 112.

As shown in Figures 2, 3A, and 3B, the first elastic belt 106 also defines an outer laterally extending edge 107a and an inner laterally extending edge 107b, and the second elastic belt 108 defines an outer laterally extending edge 109a and an inner laterally extending edge 109b. The outer laterally extending edges 107a, 109a may also define the front waist edge 121 and the laterally extending back waist edge 122. The first elastic belt and the second elastic belt may also each include an outer, garment facing layer 162 and an inner, wearer facing layer 164. It is to be appreciated that the first elastic belt 106 and the second elastic belt 108 may comprise the same materials and/or may have the same structure. In some embodiments, the first elastic belt 106 and the second elastic belt may comprise different materials and/or may have different structures. It should also be appreciated that the first elastic belt 106 and the second elastic belt 108 may be constructed from various materials. For example, the first and second belts may be manufactured from materials such as plastic films; apertured plastic films; woven or nonwoven webs of natural materials (e.g., wood or cotton fibers), synthetic fibers (e.g., polyolefins, polyamides, polyester, polyethylene, or polypropylene fibers) or a combination of natural and/or synthetic fibers; or coated woven or nonwoven webs. In some embodiments, the first and second elastic belts include a nonwoven web of synthetic fibers, and may include a stretchable nonwoven. In other embodiments, the first and second elastic belts include an inner hydrophobic, non-stretchable nonwoven material and an outer hydrophobic, non-stretchable nonwoven material.

The first and second elastic belts 106, 108 may also each include belt elastic material interposed between the outer layer 162 and the inner layer 164. The belt elastic material may include one or more elastic elements such as strands, ribbons, films, or panels extending along the lengths of the elastic belts. As shown in Figures 2, 3A, and 3B, the belt elastic material may include a plurality of elastic strands 168 which may be referred to herein as outer, waist elastics 170 and inner, waist elastics 172. Elastic strands 168, such as the outer waist elastics 170, may continuously extend laterally between the first and second opposing end regions 106a, 106b of the first elastic belt 106 and between the first and second opposing end regions 108a, 108b of the second elastic belt 108. In some embodiments, some elastic strands 168, such as the inner waist elastics 172, may be configured with discontinuities in areas, such as for example, where the first and second elastic belts 106, 108 overlap the absorbent assembly 140. In some embodiments, the elastic strands 168 may be disposed at a constant interval in the longitudinal direction. In other embodiments, the elastic strands 168 may be disposed at different intervals in the longitudinal direction. The belt elastic material in a stretched condition may be interposed and joined between the uncontracted outer layer and the uncontracted inner layer. When the belt elastic material is relaxed, the belt elastic material returns to an unstretched condition and contracts the outer layer and the inner layer. The belt elastic material may provide a desired variation of contraction force in the area of the ring-like elastic belt. It is to be appreciated that the chassis 102 and elastic belts 106, 108 may be configured in different ways other than as depicted in Figure 2. The belt elastic material may be joined to the outer and/or inner layers continuously or intermittently along the interface between the belt elastic material and the inner and/or outer belt layers.

In some configurations, the first elastic belt 106 and/or second elastic belt 108 may define curved contours. For example, as shown in Figure 2, the inner lateral edge 109b of the second elastic belt 108 may include non-linear or curved portions 109c in the first and second opposing end regions 108a, 108b. Such curved contours may help define desired shapes to leg opening 112, such as for example, relatively rounded leg openings. Although the inner lateral edge 107b of the first elastic belt is depicted as being a straight line, it is to be appreciated that the inner lateral edge 107b may also include curved portions in the first and second opposing end regions 106a, 106b. In addition to having curved contours, the elastic belts 106, 108 may include elastic strands 168, 172 that extend along non-linear or curved paths that may correspond with the curved contours of the inner lateral edges 107b, 109b.

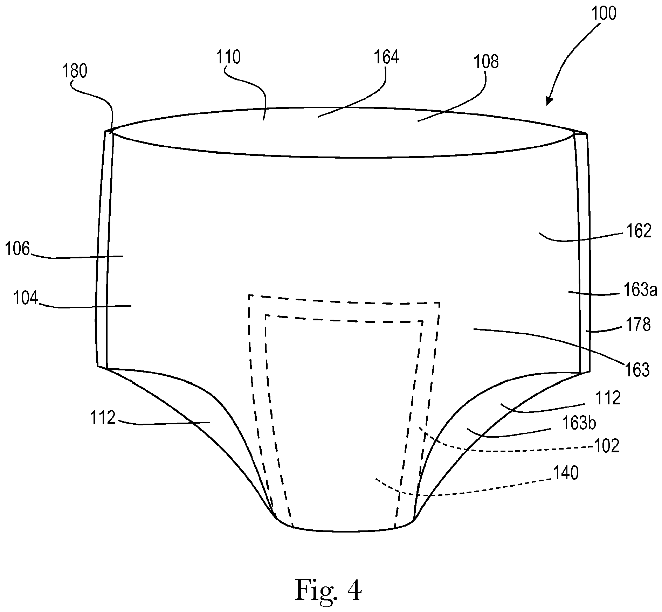

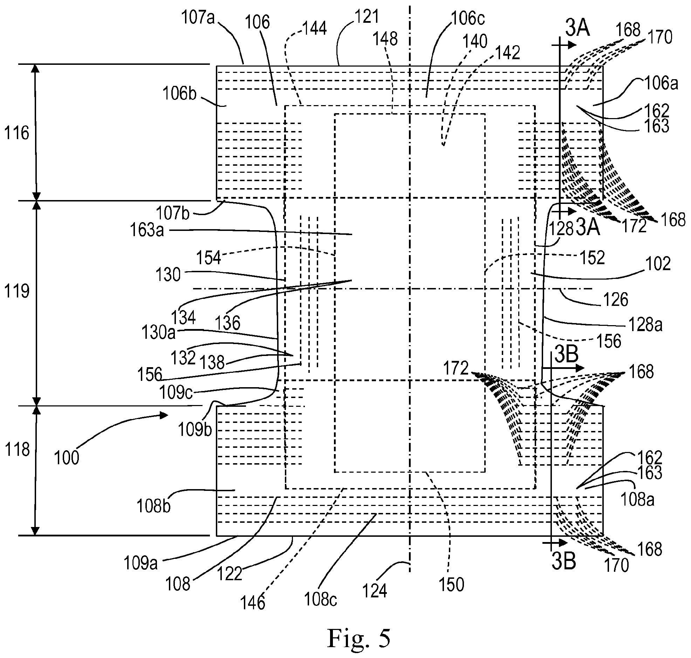

It is to be appreciated that the diaper pant 100 may include a chassis 102 and elastic belts 106, 108 configured in different ways other than as depicted in Figures 1-2B. For example, Figures 4 and 5 show a diaper pant 100 having the same components as described above with reference to Figures 1-2B, except the outer layer 162 of the elastic belt 106, 108 is a configured as a contiguous outer cover 163 that extends through the first waist region 116, crotch region 119, and second waist region 118. Thus, as shown in Figures 4 and 5, the diaper 100 the outer cover 163 also includes a first waist end region 116, a crotch region 119, and an opposing second waist end region 118. The outer cover 163 also includes a garment facing surface 163a and an opposing wearer facing surface 163b. As such, elastic members 168 of the elastic belts 106, 108 may be connected with the wearer facing surface 163b of the outer cover 163. And the chassis 102 may be positioned on the wearer facing surface 163b of the outer cover 163. As such, the backsheet 136 may include a portion of the outer cover 163. In addition, the outer cover 163 may include a first longitudinal side edge 128a and a second longitudinal side edge 130a that are positioned laterally outboard the first longitudinal side edge 128 of the chassis 102 and second longitudinal side edge 130 of the chassis 102, respectively, as shown in Figure 5. It is to be appreciated also that the first longitudinal side edge 128a and a second longitudinal side edge 130a may aligned with or positioned laterally inboard of the first longitudinal side edge 128 of the chassis 102 and second longitudinal side edge 130 of the chassis 102, respectively.

Although the following methods are provided in the context of the diapers 100 shown in Figures 1-5, it is to be appreciated that various embodiments of diaper pants can be folded and stacked according to the methods disclosed herein, such as for example, the absorbent articles disclosed in U.S. Patent Publication Nos. 2011/0167765 A1, 2013/0130879 A1, and 2013/0211636 A1.

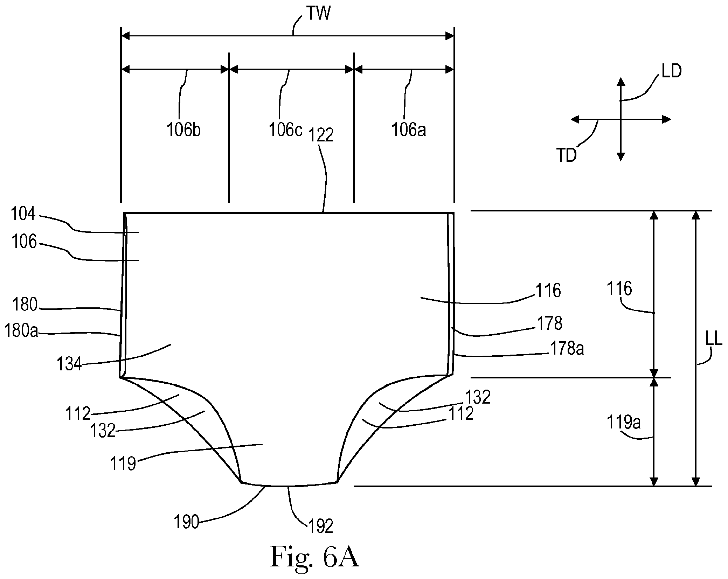

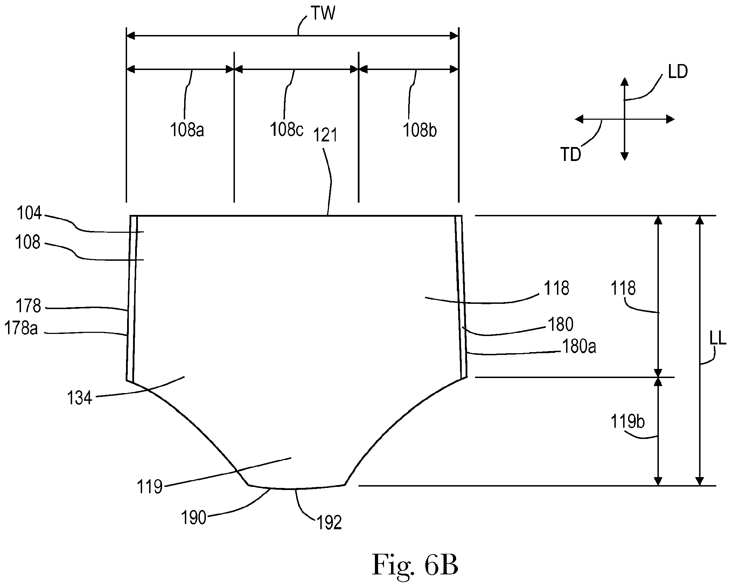

As discussed in more detail below with reference to Figures 7-9, the methods and apparatuses herein may include a folding apparatus 700 adapted to fold absorbent articles 100 and advancing the folded absorbent articles 100 to a stacker apparatus 800. The absorbent articles may be in the form of diaper pants 100 such as those embodiments discussed above with reference to Figures 1A-5. To place the following description of the methods and apparatuses in better context as relating to various regions of diaper pants 100 that may be folded. In particular, Figure 6A shows a front plan view of a diaper pant 100 in a laid flat condition illustrating various regions of the diaper pant 100. And Figure 6B shows a rear front plan view of diaper pant 100 in a laid flat condition illustrating various regions of the diaper pant 100. As discussed above, the diaper pant 100 defines include an inner, body facing surface 132, and an outer, garment facing surface 134. The diaper pant 100 also defines a longitudinal direction LD that extends from the waist edges 121, 122 to a crotch end 190 that is defined by a lateral fold line 192 in the crotch region 119. As such, the lateral fold line 192 divides the crotch region into a first crotch region 119a and a second crotch region 119b. The diaper pant 100 also defines a transverse direction TD is perpendicular to the longitudinal direction LD.

The diaper pant 100 is shown in Figures 6A and 6B as having a first elastic belt 106, and a second elastic belt 108. The first belt 106 has a first end region 106a, an opposing second end region 106b, and a central region 106c. And the second belt 108 has a first end region 108a, an opposing second end region 108b, and a central region 108c. The first end regions 106a, 108a are connected together at a first side seam 178, and the second end regions are 106b, 108b are connected together at a second side seam 180. The diaper pant also defines a transverse width TW that extends in a transverse direction TD between a first side edge 178a and a second side edge 180a, and is measured along longitudinal midpoints of each side seam 178, 180.

The first end region 106a the first belt 106 extends approximately 20% to 40% of the transverse width TW of the diaper pant 100 in an assembled, laid-flat, relaxed condition, and the first end region 108a the second belt 108 extends approximately 20% to 40% of the transverse width TW of the diaper pant 100 in an assembled, laid-flat, relaxed condition. The second end region 106b the first belt 106 and extends approximately 20% to 40% of the transverse width TW of the diaper pant 100 in an assembled, laid-flat, relaxed condition, and the second end region 108b the second belt 108 extends approximately 20% to 40% of the transverse width TW of the diaper pant 100 in an assembled, laid-flat, relaxed condition. The central region 106c the first belt 106 and extends approximately 20% to 60% of the transverse width TW of the diaper pant 100 in an assembled, laid-flat, relaxed condition, and the central region 108c the second belt 108 extends approximately 20% to 60% of the transverse width TW of the diaper pant 100 in an assembled, laid-flat, relaxed condition.

The diaper pant 100 in Figures 6A and 6B is also shown as having a longitudinal length LL that is defined by the distance between the first waist edge 121 and the crotch end 190, or if longer, the distance from the second waist edge 122 to the crotch end 190. The longitudinal length LL is measured along the longitudinal centerline 124 of the diaper pant 100. As shown in Figure 6A, the first waist region 116 extends a distance generally in the longitudinal direction LD from the waist edge 121 along the side seams 178, 180 to the leg openings 112, and the second waist region 118 extends a distance generally in the longitudinal direction LD from the waist edge 122 along the side seams 178, 180 to the leg openings 112. Hence, the first crotch region 119a extends a distance from the crotch end 190 to the first waist region 116, and the second crotch region 119b extends a distance from the crotch end 190 to the second waist region 118. In some embodiments, the first waist region 116 and/or the second waist region 118 may extend about two-thirds the longitudinal length LL of the assembled diaper pant 100. In addition, the first crotch region 119a and/or the second crotch region 119b may extend about one-third the longitudinal length LL of the assembled diaper pant 100.

Figure 7 shows a schematic side view of a folding apparatus 700 adapted to fold discrete diaper pants 100 and transfer the folded diaper pants 100 to a stacker apparatus 800. The folding system 700 may include a first carrier 702, wherein the first carrier 702 advances diaper pants 100 a first machine direction 704. A second carrier 706 is located adjacent the first carrier 702 to define a nip 708 extending in a second machine direction 710. More particularly, the first carrier 702 may be in the form of a belt conveyor including a first belt 712 defining a first carrier surface 714. As shown in Figure 7, the first belt 712 may be routed in an endless loop around rollers 716. The second carrier 706 may be in the form of belt conveyor including a second belt 718 defining a second carrier surface 720. The second belt 718 may be routed in an endless loop around rollers 716. As such, the nip 708 extends in the second machine direction 710 between the first carrier surface 714 and the second carrier surface 720. As shown in Figures 7 and 8A, the folding apparatus 700 also includes a first folding member 722 and a second folding member 724. As discussed in more detail below, the first carrier 702 advances discrete diaper pants 100 in the first machine direction 704 between the first carrier surface 702 and the first and second folding members 722, 724 to fold the first and second belts 106, 108 along longitudinally extending fold lines. The partially folded diaper pants 100 are then folded laterally and advanced by the first carrier 702 and the second carrier 706 in the second machine direction 710 through the nip 708. The folded diaper pants 100 are then transferred from the nip 708 to a third carrier 726 that reorients and advances the folded diaper pants 100 in a third machine direction 728 to the stacker apparatus 800.

As shown in Figures 7 and 8A, the first folding member 722 and/or the second folding member 724 may be configured as plates extending along the first machine direction 704 and separated from the first carrier surface 714. The first folding member 722 includes an upstream folding edge 730 and a downstream folding edge 732. And the second folding member 724 includes an upstream folding edge 734. The first folding member 722 and the second folding member 724 may be separated from each other along the first machine direction 704 to define a slot 736 between the downstream folding edge 732 and the upstream folding edge 734. As shown in Figure 8A, the folding edges 730, 732, 734 may be configured to define acute angles with respect to the first machine direction 704, and may extend in a cross direction CD across the first carrier surface 714.

In some embodiments, diaper pants 100 may assembled by a manufacturing process to resemble the diaper pants 100 shown in Figures 6A and 6B. Once assembled, the diaper pants 100 may advance to a folding apparatus 700, such as shown in Figure 7. With particular reference to Figures 7, 8A, and 8A1, the diaper pants 100 may be positioned on first carrier surface 714 with first waist edge 121 and the second waist edge 122 extending in the cross direction CD and the crotch end 190 positioned downstream of the waist edges 121, 122. In some configurations, the diaper pant 100 may be positioned on the first carrier surface 702 such that the second waist region 118 and the second crotch region 119b are in a facing relationship with the first carrier surface 714. Thus, the first carrier 702 advances the diaper pant 100 in the first machine direction 704 on the first carrier surface 714 from location 8A1 and in the orientation shown in Figure 8A1. The diaper pant 100 then advances from location 8A1 to where the first end regions 106a, 108a of the first belt 106 and second belt 108 contact the upstream folding edge 703 of the first folding member 722. As the diaper pant 100 continues to advance in the first machine direction 704 between the first folding member 722 and the first carrier surface 714 to location 8A2, the upstream folding edge 730 of the first folding member 722 directs the first end regions 106a, 108a of the first belt 106 and second belt 108 to be folded over the central region 106c of the first belt 106. Thus, as shown in Figure 8A2, the diaper pant 100 is folded along a longitudinally extending first fold line 900 to position the first end region 106a of the first elastic belt 106 in a facing relationship with the central region 106c of the first elastic belt 106.

As shown in Figure 8A2, a protruding portion 901 of the diaper pant 100 may extend beyond the first fold line 900. The protruding portion 901 example is illustrated in Figure 8A2 as being a generally fin-shaped extension. The protruding portion 901 may be defined by a portion of a substrate of the diaper pant 100, such as a portion of the outer cover 163 for example, that is not folded along the first fold line 900 or may have been subsequently pulled from the first fold line 900 by forces exerted on the diaper pant 100. As previously mentioned, such a protruding portion 901 may be formed as a result of forces exerted on the diaper pant 100 as a result of the relatively high travel speeds, friction from various components of unit operation apparatuses, and/or elasticity of the diaper pant components. As discussed in more detail below, the folding apparatus 700 can be configured to orient and advance the folded diaper pants 100 so as to help reduce the likelihood that the protruding portions 901 will unintentionally interfere with the advancement of the diaper pants 100 to the stacker apparatus 800.

From position 8A2 on the first carrier 702, the diaper pant 100 advances in the first machine direction 704 to where the second end regions 106b, 108b of the first belt 106 and second belt 108 contact the upstream folding edge 734 of the second folding member 724. As the diaper pant 100 continues to advance in the first machine direction 704 between the second folding member 724 and the first carrier surface 714, the upstream folding edge 734 of the second folding member 724 directs the second end regions 106b, 108b of the first belt 106 and second belt 108 to be folded over the central region 106c of the first belt 106. As such, the second end regions 106b, 108b of the first belt 106 and second belt 108 are directed along the slot 736 between the first and second folding members 722, 724. As shown in the transition from Figure 8A2 to Figure 8A3, the diaper pant 100 is folded along a longitudinally extending second fold line 902 to position the second end region 106b of the first elastic belt 106 in a facing relationship with the first end region 108a of the second elastic belt 108.

With reference to Figures 7, 8A, 8A2, and 8A3, the diaper pant 100 continues to advance in the first machine direction 704 from location 8A3 to the nip 708, the diaper pant 100 is oriented such that the second end region 108b of the second elastic belt 108 is in a facing relationship with and in contact with a stationary surface 725 of the second folding member 724. And the central region 108c of the second elastic belt 108 is in a facing relationship with and in contact with the advancing first carrier surface 714. As such, the second end region 108b of the second elastic belt 108 is forced across the central region 106c of the first elastic belt 106 due to friction from the second folding member 724. In some embodiments, friction forces created by contact between the second end region 108b of the second elastic belt 108 and the stationary surface 725 forces the second end region 108b of the second elastic belt 108 across the central region 106c of the first elastic belt 106 such that the second fold line 902 extends along and is partially defined by a longitudinal edge 154 of the absorbent core 140. It should also be appreciated that in some embodiments, the first fold line 900 may extend along and/or may be partially defined by a longitudinal edge 152 of the absorbent core 140.

As shown in Figure 7, the folding apparatus 700 may include one or more tucker blades 738 that are configured to engage diaper pants 100 advancing on the first carrier 702 past the nip 708. In turn, the diaper pants 100 are folded while being forced in the second machine direction 710 and into the nip 708 by the tucker blade. For example, from location 8A3 shown in Figure 7, the first carrier 702 conveys the partially folded diaper pants 100 such that the crotch end 190 of each diaper pant 100 advances in the first machine direction 704 past the nip 710 and the tucker blade 738. The tucker blade 738 is moved into position to contact the diaper pant 100 in either the first waist region 116 or crotch region 119 and redirect the diaper pant 100 into the nip 708. As the diaper pant 100 is directed in to the nip 708, the first crotch region 119a is positioned in a facing relationship with the second end region 108b of the second elastic belt 108, thereby creating a laterally extending third fold line 904 to form a folded absorbent article 100, such as shown in Figure 8B. As the folded articles 100 advance through the nip 708, the second waist region 118 is in facing relationship with the first carrier surface 714 and the second crotch region 119b is in a facing relationship with the second carrier surface 720. It is to be appreciated that the folding system 700 may be configured such that the tucker blades 738 contact the diaper pants 100 so as create third fold lines 904 that are equidistant from the first waist edge 121 and the crotch end 190, or equidistant from the second waist end 122 and the crotch end 190. As such, in some configurations, the third fold lines 904 may be positioned so as to bisect the article 100. It is also to be appreciated that the tucker blades 738 may be configured such that the third fold line 904 may be positioned in various locations other than the geometric center of the diaper pants 100. In some configurations, the third fold line 904 may be extend along and/or may be partially defined by a lateral front edge 148 and/or lateral back 150 of the absorbent core 140. In some configurations, the absorbent core 140 may also be folded when creating the third fold line 904. For example, the third fold line 904 may extend across one end region or both opposing end regions of the absorbent core 140.

As shown in Figure 7, a drive mechanism 740 may be connected with the tucker blade 738 to move the tucker blade 738 into contact with the advancing diaper pants 100. It is to be appreciated that the drive mechanism 740 may be configured to impart various types of motion to the tucker blades 738. For example, the drive mechanism 740 may impart a reciprocating motion to the tucker blade 738, such as an up and down (or back and forth) reciprocating motion. In some embodiments, the drive mechanism 740 may be adapted to rotate the tucker blades 738 into engagement with the advancing diaper pants 100. In some configurations, the drive mechanisms 740 may include one or more motors directly or indirectly connected with one or more tucker blades 738. In some configurations, a motor may be connected with the tucker blades through various types of transmission, belt, and/or gear arrangements. In addition, the motors may be configured as servo motors that may operate with constant or variable speeds. Various examples of drive mechanism arrangements that may be used to rotate the tucker blades are disclosed in U.S. Patent No. 7,617,656. It is to be appreciated that the drive mechanism may be configured to rotate the tucker blades at a constant angular velocity. In some embodiments, the angular velocity of the tucker blades may be varied on a cyclic basis with a drive mechanism that may include a servo motor. It is to be appreciated that the folding system 700 may be configured with various numbers of tucker blades, such as disclosed in for example in U.S. Patent Application Nos. 61/824,003 and 61/824,013.

With continued reference to Figure 7, the folded diaper pants 100 advance in the second machine direction 710 through the nip 708 between the first and second carriers 702, 706. From the nip 708, the folded diaper pants 100 advance to the third carrier 726 that reorients the folded diaper pants 100 before being transferred to the stacker apparatus 800. The third carrier 726 may include a first twisted conveyor belt 742 and a second twisted conveyor belt 744. As shown in Figures 7 and 8C, the folded diaper pants 100 may be positioned between the first and second twisted conveyor belts 742, 744 such that the first twisted belt 742 is in a facing relationship with the second waist region 118, and the second twisted belt 744 is in a facing relationship with the second crotch region 199b. In addition, the third fold line 904 is positioned downstream of the waist edges 121, 122 and crotch end 190. As also shown in Figures 7 and 8C, the folded diaper pants 100 may be oriented in a generally horizontal position wherein the second crotch region 119b is above the second waist region 118. With reference to Figures 7, 8C, 8D, and 8E, the first and second twisted belts 742, 744 advance the folded diaper pants 100 in a third machine direction 728 while reorienting the diapers 100 into a generally vertical orientation wherein the second fold line 902 is below the first fold line 900. In turn, the protruding portion 901 of the folded diaper 100 will also be positioned above the second fold line 902.

As previously mentioned, the folded diaper pants 100 advance from the third carrier 726 to the stacker apparatus 800. As shown in Figures 7, 8D, 8F, and 8G the stacker apparatus 800 includes receptacles 802 that receive the folded diaper pants 100 from the first and second twisted conveyor belts 742, 744. The receptacles 802 are also advanced in the cross direction CD relative to third machine direction 728 defined by the first and second twisted conveyor belts 742, 744. As such, once an empty receptacle 802 receives a folded diaper pant 100, the receptacle 802 and folded diaper pant 100 are advanced in the cross direction CD to position an adjacent empty receptacle 800 at the discharge or downstream ends of the first and second twisted conveyors 742, 744 to receive a subsequently advancing folded diaper pant 100. The sequence is repeated to fill a plurality of receptacles with folded absorbent articles 100, such as shown in Figure 9. Thus, a plurality of folded absorbent articles 100 are advanced in as a stack 950 into a package 952.

Referring back to the Figures 7, 8D, 8F, and 8G each receptacle 802 is defined by a bottom wall 804, a rear wall 806, a first side member 808, and a second side member 810 spaced apart from the first side member 808 in a cross direction CD, wherein the first side member 808 and the second side member 810 are both connected with the rear wall 806. The bottom wall 804 may be configured a stationary surface. And the rear wall 806, first side member 808, and second side member 810 may all be configured to advance in the cross direction CD relative to the bottom wall 806. As shown in Figures 7 and 8D, the stacker apparatus 800 is positioned downstream of third carrier 726 to define a gap 812 between downstream ends of the first and second twisted conveyors 742, 744 and the bottom wall 804 of the receptacles 802. By orienting each folded diaper 100 on the third carrier 726 such that the protruding portion 901 of the folded diaper 100 is positioned above the second fold line 902, the second fold line 902 of the folded diapers 100 advances across the gap 812. And because the protruding portion 901 of the folded diaper 100 does not advance across the gap 812 and because the protruding portion 901 is not adjacent the bottom wall 804, the likelihood of protruding portion 901 of the diapers 100 interfering with the advancement into the receptacles 802 is reduced. As shown in Figures 7, 8D, 8E, 8F, and 8G the folded absorbent articles 100 are inserted into the stacker receptacles 802 such that the second fold line 902 is in contact with the bottom wall 804. In addition, the central region 108c of the second elastic belt 108 is in a facing relationship with the first side member 808, and the second crotch region 119b is in a facing relationship with second side member 810. In addition, the first fold line 900 is above the second fold line 902. Once the absorbent article 100 is inserted into the receptacle 802, the first side member 808, the second side member 810, the rear wall 806, and the folded absorbent article 100 are advanced in the cross direction CD.

It is to be appreciated that additional folding configurations can be utilized with the folding and stacking apparatuses and methods herein. For example, as shown in Figures 10A1-10A4 illustrate another embodiment of a folding sequence of a diaper pant 100. As shown in Figures 10A1, 10A2, and 10A3, the first elastic belt 106 and the second elastic belt 108 are first folded along longitudinally extending belt fold lines 906 to position a distal portion 108d of the first end region 108a of the second elastic belt 108 in a facing relationship with a proximal portion 108p of the first end region 108a of the second elastic belt 108 and to position a distal portion 108d of the second end region 108b of the second elastic belt 108 in a facing relationship with a proximal portion 108p of the second end region 108b of the second elastic belt 108. As shown in Figures 10A3 and 10A4, the absorbent article 100 may then be folded along a longitudinally extending first fold line 900 to position a proximal portion 106p of the first end region 106a of the first elastic belt 106 in a facing relationship with the central region 106c of the first elastic belt 106. With reference to Figures 10A4 and 10A5, the absorbent article 100 may then be folded along a longitudinally extending second fold line 902 to position a proximal portion 106p of the second end region 106b of the first elastic belt 106 in a facing relationship with central region 106c of the first elastic belt 106. As shown in Figure 10A5, the proximal portion 106p of the second end region 106b of the first elastic belt 106 may also be placed to overlap the distal portion 108d of the first end region 108a of the second elastic belt 108. As shown in Figures 10A4 and 10A5 the folded diaper 100 may also include a protruding portion 901 extending beyond the first fold line 900, such as discussed above with reference to Figures 8A2 and 8A3.

Once partially folded into the configuration shown in Figure 10A5, the diaper pant 100 may then be folded along a laterally extending third fold line 904 such as described above with reference to Figures 7 and 8B. As such, folding the article 100 along the third fold line 904 positions the crotch region 119 in a facing relationship with the first end region 106a of the first elastic belt 106 and the second end region 106b of the first elastic belt 106 to form a folded absorbent article. As discussed above, the folded diaper pant 100 may then be oriented such that the second fold line 902 is below the first fold line 900. And the folded absorbent article 100 may be advanced in a machine direction and inserted into stacker receptacle 802 and subsequently packaged, such as described above with reference to Figures 7-9. Thus, the diaper pant 100 may be inserted into a receptacle 802 such that the second fold line 902 is in contact with the bottom wall 804, wherein the central region 108c of the second elastic belt 108 is in a facing relationship with the first side member 808 and wherein the crotch region 119 is in a facing relationship with second side member 810, wherein the first fold line 900 is above the second fold line 902.

It is to be appreciated that the methods of assembly, folding, stacking, and packaging of diaper pants specifically described herein and illustrated in the accompanying drawings are non-limiting example embodiments. The features illustrated or described in connection with one non-limiting embodiment may be combined with the features of other non-limiting embodiments. Such modifications and variations are intended to be included within the scope of the present disclosure.

## Claims

1. A method for making an absorbent product comprising a package (952) of absorbent articles (100), each absorbent article (100) having a longitudinal axis (124) and a lateral axis (126) and comprising: a first elastic belt (106), a second elastic belt (108), and a chassis (102) extending longitudinally between the first elastic belt (106) and the second elastic belt (108); wherein the chassis (102) comprises a topsheet (138), a backsheet (136), and an absorbent core (140) disposed between the topsheet (138) and the backsheet (136); wherein the first and second elastic belts (106, 108) each comprise a first end region (106a, 108a) and a second end region (106b, 108b) separated by a central region (106c, 108c); and wherein the first end region (106a) of the first elastic belt (106) is connected with the first end region (108a) of the second elastic belt (108) at a first side seam (178), and the second end region (106b) of the first elastic belt (106) is connected with the second end region (108b) of the second elastic belt (108) at a second side seam (180) to define a continuous perimeter waist opening (110) and two continuous perimeter leg openings (112), wherein the first elastic belt (106) defines a first waist region (116), the second elastic belt (108) defines a second waist region (118), the first and second waist regions (116, 118) connected with each other by a crotch region (119), the method comprising the steps of:
folding the absorbent article (100) along a longitudinally extending first fold line (900) to position the first end region (106a) of the first elastic belt (106) in a facing relationship with the central region (106c) of the first elastic belt (106);
folding the absorbent article (100) along a longitudinally extending second fold line (902) to position the second end region (106b) of the first elastic belt (106) in a facing relationship with the first end region (108a) of the second elastic belt (108);
forcing the second end region (108b) of the second elastic belt (108) across the central region (106c) of the first elastic belt (106);
folding the absorbent article (100) along a laterally extending third fold line (904) to position the crotch region (119) in a facing relationship with the first end region (108a) of the second elastic belt (108) and the second end region (108b) of the second elastic belt (108) to form a folded absorbent article (100);
orienting the folded absorbent article (100) such that the second fold line (902) is below the first fold line (900);
advancing the folded absorbent article (100) in a machine direction to a stacker receptacle (802), the stacker receptacle comprising: a bottom wall (804), a rear wall (806), a first side member (808), and a second side member (810) spaced apart from the first side member (808) in a cross direction, wherein the first side member (808) and the second side member (810) are both connected with the rear wall (806);
inserting the folded absorbent article (100) into the stacker receptacle (802) such that the second fold line (902) is in contact with the bottom wall (804), and wherein the central region (108c) of the second elastic belt (108) is in a facing relationship with the first side member (808) and wherein the crotch region (119) is in a facing relationship with second side member (810), wherein the first fold line (900) is above the second fold line (902), and wherein the third fold line (904) is placed in contact with the rear wall (806) of the receptacle (802);
moving the first side member (808), the second side member (810), and the folded absorbent article (100) in the cross direction; and
advancing the folded absorbent article (100) from the receptacle to the package (952).

2. The method of claim 1, wherein the step of folding the absorbent article (100 along a laterally extending third fold line (904) further comprises folding an end region of the absorbent core (140).

3. The method according to any of the preceding claims, wherein the step of forcing the second end region (108b) of the second elastic belt (108) across the central region (106c) of the first elastic belt (106) further comprises advancing the absorbent article (100) between a moving carrier surface (714) and a stationary surface (725), wherein the central region (108c) of the second elastic belt (108) is in a facing relationship with the moving carrier surface (714) and the second end region (108b) of the second elastic belt (108) is in facing relationship with the stationary surface (725).

4. The method of claim 3, wherein friction created by contact between the second end region (108b) of the second elastic belt (108) and the stationary surface (725) forces the second end region (108b) of the second elastic belt (108) across the central region (106c) of the first elastic belt (106) such that the second fold line (902) extends along a longitudinal edge of the absorbent core (140).

5. The method of claim 3, wherein the moving carrier surface (714) comprises an endless belt (712).

6. The method according to any of the preceding claims, wherein the absorbent article comprises an outer cover (163) comprising a first waist end region (116) and an opposing second waist end region (118), and having garment facing surface (163a) and an opposing wearer facing surface (163b), wherein the first elastic belt (106) is positioned in the first waist end region (116) and the second elastic belt (108) is positioned in the second waist end region (118).

7. The method of claim 6, wherein the first elastic belt (106) comprises an elastic member (168) connected with the wearer facing surface (163b) of the outer cover (163).

8. The method of claim 7, wherein the chassis (102) is positioned on the wearer facing surface (163b) of the outer cover (163).

9. The method of claim 8, wherein the backsheet (136) comprises a portion of the outer cover (163).

## Patentansprüche

1. Verfahren zur Herstellung eines Absorptionsprodukts, umfassend eine Verpackung (952) von Absorptionsartikeln (100), wobei jeder Absorptionsartikel (100) eine Längsachse (124) und eine Querachse (126) aufweist und umfasst: einen ersten Gummibandbund (106), einen zweiten Gummibandbund (108) und eine Grundeinheit (102), die sich in Längsrichtung zwischen dem ersten Gummibandbund (106) und dem zweiten Gummibandbund (108) erstreckt; wobei die Grundeinheit (102) eine Oberschicht (138), eine Unterschicht (136) und einen Absorptionskern (140) umfasst, der zwischen der Oberschicht (138) und der Unterschicht (136) angeordnet ist; wobei der erste und der zweite Gummibandbund (106, 108) jeweils einen ersten Endbereich (106a, 108a) und einen zweiten Endbereich (106b, 108b) umfassen, die durch einen Mittelbereich (106c, 108c) getrennt sind; und wobei der erste Endbereich (106a) des ersten Gummibandbundes (106) mit dem ersten Endbereich (108a) des zweiten Gummibandbundes (108) an einer ersten Seitennaht (178) verbunden ist und der zweite Endbereich (106b) des ersten Gummibandbundes (106) mit dem zweiten Endbereich (108b) des zweiten Gummibandbundes (108) an einer zweiten Seitennaht (180) verbunden ist, um eine Taillenöffnung mit ununterbrochenem Umfang (110) und zwei Beinöffnungen mit ununterbrochenem Umfang (112) zu bestimmen, wobei der erste Gummibandbund (106) einen ersten Taillenbereich (116) bestimmt, der zweite Gummibandbund (108) einen zweiten Taillenbereich (118) bestimmt, die ersten und zweiten Taillenbereiche (116, 118) miteinander durch einen Schrittbereich (119) verbunden sind, das Verfahren umfassend die Schritte:
Falten des Absorptionsartikels (100) entlang einer sich in Längsrichtung erstreckenden ersten Faltlinie (900), um den ersten Endbereich (106a) des ersten Gummibandbundes (106) in einer einander zugewandten Beziehung mit dem Mittelbereich (106c) des ersten Gummibandbundes (106) anzuordnen;
Falten des Absorptionsartikels (100) entlang einer sich in Längsrichtung erstreckenden zweiten Faltlinie (902), um den zweiten Endbereich (106b) des ersten Gummibandbundes (106) in einer einander zugewandten Beziehung mit dem ersten Endbereich (108a) des zweiten Gummibandbundes (108) anzuordnen;
Drängen des zweiten Endbereichs (108b) des zweiten Gummibandbundes (108) über den Mittelbereich (106c) des ersten Gummibandbundes (106) hinweg;
Falten des Absorptionsartikels (100) entlang einer sich seitlich erstreckenden dritten Faltlinie (904), um den Schrittbereich (119) in einer einander zugewandten Beziehung mit dem ersten Endbereich (108a) des zweiten Gummibandbundes (108) und dem zweiten Endbereich (108b) des zweiten Gummibandbundes (108) anzuordnen, um einen gefalteten Absorptionsartikel (100) zu bilden;
Ausrichten des gefalteten Absorptionsartikels (100) auf derartige Weise, dass die zweite Faltlinie (902) unter der ersten Faltlinie (900) liegt;
Vorwärtsbewegen des gefalteten Absorptionsartikels (100) in eine Maschinenrichtung zu einem Stapelbehälter (802), wobei der Stapelbehälter umfasst: eine Bodenwand (804), eine hintere Wand (806), ein erstes Seitenelement (808), und ein zweites Seitenelement (810), das von dem ersten Seitenelement (808) in einer Querrichtung beabstandet ist, wobei das erste Seitenelement (808) und das zweite Seitenelement (810) beide mit der hinteren Wand (806) verbunden sind;
Einsetzen des gefalteten Absorptionsartikels (100) in den Stapelbehälter (802) auf derartige Weise, dass die zweite Faltlinie (902) in Kontakt mit der Bodenwand (804) steht, und wobei der Mittelbereich (108c) des zweiten Gummibandbundes (108) in einer einander zugewandten Beziehung mit dem ersten Seitenelement (808) steht, und wobei der Schrittbereich (119) in einer einander zugewandten Beziehung mit dem zweiten Seitenelement (810) steht, wobei die erste Faltlinie (900) oberhalb der zweiten Faltlinie (902) liegt und wobei die dritte Faltlinie (904) in Kontakt mit der hinteren Wand (806) des Behälters (802) gebracht wird;
Bewegen des ersten Seitenelements (808), des zweiten Seitenelements (810) und des gefalteten Absorptionsartikels (100) in die Querrichtung; und
Vorwärtsbewegen des gefalteten Absorptionsartikels (100) von dem Behälter zu der Verpackung (952).

2. Verfahren nach Anspruch 1, wobei der Schritt des Faltens des Absorptionsartikels (100) entlang einer sich seitlich erstreckenden dritten Faltlinie (904) ferner das Falten eines Endbereichs des Absorptionskerns (140) umfasst.

3. Verfahren nach einem der vorstehenden Ansprüche, wobei der Schritt des Drängens des zweiten Endbereichs (108b) des zweiten Gummibandbundes (108) über den Mittelbereich (106c) des ersten Gummibandbundes (106) hinweg ferner ein Vorwärtsbewegen des Absorptionsartikels (100) zwischen einer sich bewegenden Trägeroberfläche (714) und einer stationären Oberfläche (725) umfasst, wobei der Mittelbereich (108c) des zweiten Gummibandbundes (108) in einer einander zugewandten Beziehung mit der beweglichen Trägeroberfläche (714) steht und der zweite Endbereich (108b) des zweiten Gummibandbundes (108) in einer einander zugewandten Beziehung mit der stationären Oberfläche (725) steht.

4. Verfahren nach Anspruch 3, wobei Reibung, die durch den Kontakt zwischen dem zweiten Endbereich (108b) des zweiten Gummibandbundes (108) und der stationären Oberfläche (725) erzeugt wird, den zweiten Endbereich (108b) des zweiten Gummibandbundes (108) über den Mittelbereich (106c) des ersten Gummibandbundes (106) auf derartige Weise drängt, dass sich die zweite Faltlinie (902) entlang einem Längsrand des Absorptionskerns (140) erstreckt.

5. Verfahren nach Anspruch 3, wobei die bewegliche Trägeroberfläche (714) einen Endlosbund (712) umfasst.

6. Verfahren nach einem der vorstehenden Ansprüche, wobei der Absorptionsartikel einen Außenmantel (163) umfasst, umfassend einen ersten Taillenendbereich (116) und einen gegenüberliegenden zweiten Taillenendbereich (118), und mit einer bekleidungsseitigen Oberfläche (163a) und einer gegenüberliegenden trägerseitigen Oberfläche (163b), wobei der erste Gummibandbund (106) in dem ersten Taillenendbereich (116) angeordnet ist und der zweite Gummibandbund (108) in dem zweiten Taillenendbereich (118) angeordnet ist.

7. Verfahren nach Anspruch 6, wobei erste Gummibandbund (106) ein Gummibandelement (168) umfasst, das mit der trägerseitigen Oberfläche (163b) des Außenmantels (163) verbunden ist.

8. Verfahren nach Anspruch 7, wobei die Grundeinheit (102) auf der trägerseitigen Oberfläche (163b) des Außenmantels (163) angeordnet ist.

9. Verfahren nach Anspruch 8, wobei die Unterschicht (136) einen Abschnitt des Außenmantels (163) umfasst.

## Revendications

1. Procédé de fabrication d'un produit absorbant comprenant un conditionnement (952) d'articles absorbants (100), chaque article absorbant (100) ayant un axe longitudinal (124) et un axe latéral (126) et comprenant : une première ceinture élastique (106), une deuxième ceinture élastique (108) et un châssis (102) s'étendant longitudinalement entre la première ceinture élastique (106) et la deuxième ceinture élastique (108) ; dans lequel le châssis (102) comprend une feuille de dessus (138), une feuille de fond (136) et une âme absorbante (140) disposée entre la feuille de dessus (138) et la feuille de fond (136) ; dans lequel les première et deuxième ceintures élastiques (106, 108) comprennent chacune une première région d'extrémité (106a, 108a) et une deuxième région d'extrémité (106b, 108b) séparées par une région centrale (106c, 108c) ; et dans lequel la première région d'extrémité (106a) de la première ceinture élastique (106) est reliée à la première région d'extrémité (108a) de la deuxième ceinture élastique (108) au niveau d'une première jonction latérale (178), et la deuxième région d'extrémité (106b) de la première ceinture élastique (106) est reliée à la deuxième région d'extrémité (108b) de la deuxième ceinture élastique (108) au niveau d'une deuxième jonction latérale (180) pour définir une ouverture de taille périmétrique continue (110) et deux ouvertures de jambe périmétriques continues (112), dans lequel la première ceinture élastique (106) définit une première région de taille (116), la deuxième ceinture élastique (108) définit une deuxième région de taille (118), les première et deuxième régions de taille (116, 118) reliées l'une à l'autre par une région d'entrejambe (119), le procédé comprenant les étapes consistant à :
plier l'article absorbant (100) le long d'une première ligne de pliage s'étendant longitudinalement (900) pour positionner la première région d'extrémité (106a) de la première ceinture élastique (106) dans une relation faisant face avec la région centrale (106c) de la première ceinture élastique (106) ;
plier l'article absorbant (100) le long d'une deuxième ligne de pliage s'étendant longitudinalement (902) pour positionner la deuxième région d'extrémité (106b) de la première ceinture élastique (106) dans une relation faisant face avec la première région d'extrémité (108a) de la deuxième ceinture élastique (108) ;
forcer la deuxième région d'extrémité (108b) de la deuxième ceinture élastique (108) à travers la région centrale (106c) de la première ceinture élastique (106) ;
plier l'article absorbant (100) le long d'une troisième ligne de pliage s'étendant latéralement (904) pour positionner la région d'entrejambe (119) dans une relation faisant face avec la première région d'extrémité (108a) de la deuxième ceinture élastique (108) et la deuxième région d'extrémité (108b) de la deuxième ceinture élastique (108) pour former un article absorbant plié (100) ;
orienter l'article absorbant plié (100) de telle sorte que la deuxième ligne de pliage (902) est en dessous de la première ligne de pliage (900) ;
faire avancer l'article absorbant plié (100) dans une direction machine vers un réceptacle d'empileuse (802), le réceptacle d'empileuse comprenant : une paroi inférieure (804), une paroi arrière (806), un premier élément latéral (808) et un deuxième élément latéral (810) espacé du premier élément latéral (808) dans une direction croisée, dans lequel le premier élément latéral (808) et le deuxième élément latéral (810) sont l'un et l'autre reliés à la paroi arrière (806) ;
insérer l'article absorbant plié (100) dans le réceptacle d'empileuse (802) de telle sorte que la deuxième ligne de pliage (902) est en contact avec la paroi inférieure (804), et dans lequel la région centrale (108c) de la deuxième ceinture élastique (108) est dans une relation faisant face avec le premier élément latéral (808) et dans lequel la région d'entrejambe (119) est dans une relation faisant face avec le deuxième élément latéral (810), dans lequel la première ligne de pliage (900) est au-dessus de la deuxième ligne de pliage (902), et dans lequel la troisième ligne de pliage (904) est placée en contact avec la paroi arrière (806) du réceptacle (802) ;
déplacer le premier élément latéral (808), le deuxième élément latéral (810) et l'article absorbant plié (100) dans la direction croisée ; et
faire avancer l'article absorbant plié (100) depuis le réceptacle vers le conditionnement (952).

2. Procédé selon la revendication 1, dans lequel l'étape de pliage de l'article absorbant (100 le long d'une troisième ligne de pliage s'étendant latéralement (904) comprend en outre le pliage d'une région d'extrémité de l'âme absorbante (140).

3. Procédé selon l'une quelconque des revendications précédentes, dans lequel l'étape consistant à forcer la deuxième région d'extrémité (108b) de la deuxième ceinture élastique (108) à travers la région centrale (106c) de la première ceinture élastique (106) comprend en outre le fait de faire avancer l'article absorbant (100) entre une surface de transporteur en mouvement (714) et une surface fixe (725), dans lequel la région centrale (108c) de la deuxième ceinture élastique (108) est dans une relation faisant face avec la surface de transporteur en mouvement (714) et la deuxième région d'extrémité (108b) de la deuxième ceinture élastique (108) est en relation faisant face avec la surface fixe (725).

4. Procédé selon la revendication 3, dans lequel le frottement créé par un contact entre la deuxième région d'extrémité (108b) de la deuxième ceinture élastique (108) et la surface fixe (725) force la deuxième région d'extrémité (108b) de la deuxième ceinture élastique (108) à travers la région centrale (106c) de la première ceinture élastique (106) de telle sorte que la deuxième ligne de pliage (902) s'étend le long d'un bord longitudinal de l'âme absorbante (140).

5. Procédé selon la revendication 3, dans lequel la surface de transporteur en mouvement (714) comprend une bande sans fin (712).

6. Procédé selon l'une quelconque des revendications précédentes, dans lequel l'article absorbant comprend une couverture externe (163) comprenant une première région d'extrémité de taille (116) et une deuxième région d'extrémité de taille opposée (118), et ayant une surface tournée vers le vêtement (163a) et une surface opposée tournée vers le porteur (163b), dans lequel la première ceinture élastique (106) est positionnée dans la première région d'extrémité de taille (116) et la deuxième ceinture élastique (108) est positionnée dans la deuxième région d'extrémité de taille (118).

7. Procédé selon la revendication 6, dans lequel la première ceinture élastique (106) comprend un élément élastique (168) relié à la surface tournée vers le porteur (163b) de la couverture externe (163).

8. Procédé selon la revendication 7, dans lequel le châssis (102) est positionné sur la surface tournée vers le porteur (163b) de la couverture externe (163).

9. Procédé selon la revendication 8, dans lequel la feuille de fond (136) comprend une partie de la couverture externe (163).
